# EUROPÄISCHE PATENTSCHRIFT

(11) **EP 3 258 101 B1**
(45) Veröffentlichungstag und Bekanntmachung des Hinweises auf die Patenterteilung: **11.09.2019**
(21) Anmeldenummer: 16174463.6
(22) Anmeldetag: 14.06.2016
(51) Int. Cl.: F04B 1/14, F04B 1/18, F04B 53/00, F04B 53/10, F04B 53/16, F04B 53/22, A61B 17/3203

(54) **PUMPMODUL UND VORRICHTUNG ZUR ERZEUGUNG EINES FLÜSSIGKEITSSTRAHLS**
PUMP MODULE AND DEVICE FOR PRODUCING A FLUID JET
MODULE DE POMPE ET DISPOSITIF DE PRODUCTION D'UN JET DE LIQUIDE

(43) Veröffentlichungstag der Anmeldung: 20.12.2017
(73) Patentinhaber: MEDAXIS AG, 6340 Baar (CH)
(72) Erfinder: WIDMER, Beat, 6005 Luzern (CH); GOOD, Roman, 8048 Zürich (CH); CHRISTEN, Lukas, 6004 Luzern (CH); BÜTLER, Martin, 6276 Hohenrain (CH); NAPOLETANO, Daniel, 8193 Eglisau (CH); MOSER, Beat, 8926 Uerzlikon (CH); ZWEIFEL, Adrian, 8645 Jona (CH)
(74) Vertreter: Grünecker Patent- und Rechtsanwälte PartG mbB

(56) Entgegenhaltungen:
- WO-A1-2008/086950
- DE-A1- 10 160 168
- DE-A1-102006 053 609
- DE-U1- 29 611 935

## Beschreibung

Die vorliegende Erfindung betrifft ein Pumpmodul zur Erzeugung eines Flüssigkeitsstrahls mit den oberbegrifflichen Merkmalen von Anspruch 1. Ein solches Pumpmodul ist aus der DE 101 60 168 A1 bekannt. Die vorliegende Erfindung betrifft weiter eine Vorrichtung zur Erzeugung eines Flüssigkeitsstrahls, in der das erfindungsgemäße Pumpmodul als Teil der Vorrichtung zum Einsatz kommt.

Die vorliegende Erfindung will insbesondere ein Pumpmodul angeben, welches als Verbrauchsteil zur Anmeldung kommt. Dabei soll das erfindungsgemäße Pumpmodul sich insbesondere für das Debridement mittels Wasserstrahl eignen. Bei dieser Behandlung wird ein Wasserstrahl auf eine Wunde gerichtet, um die Wunde zu reinigen und beispielsweise Schorf zu entfernen. Durch Debridement wird seit jeher die Wundheilung verbessert.

Ein Pumpmodul mit einem Pumpengehäuse, in dem zumindest ein Pumpkolben reversierend beweglich gelagert ist, der mit zumindest einem Dichtelement versehen ist, das im Pumpbetrieb dichtend in dem Pumpengehäuse aufgenommen ist, ist beispielsweise aus der US 2014/0079580 A1 bekannt. Weitere aus dem Stand der Technik bekannte Pumpmodule für das Debridement mittels Flüssigkeitsstrahl sind beispielsweise aus der US 2011/0150680 A1, der US 2002/0176788 A1 oder der US 2010/0049228 A1 bekannt. Dabei lassen bereits diese Belege aus dem Stand der Technik das Bemühen der Fachwelt erkennen, ein Pumpmodul anzugeben, welches lösbar mit einem Antrieb zu verbinden ist, um eine Vorrichtung zur Erzeugung eines Flüssigkeitsstrahls zu schaffen, bei welcher das Modul das Verbrauchsteil ist. So ist das Pumpmodul relativ einfach und kostengünstig aufgebaut.

Auch der vorliegenden Erfindung liegt das Problem zugrunde, ein Pumpmodul der eingangs genannten Art vorzuschlagen, welches sich einfach herstellen lässt, gleichwohl aber die für den Pumpbetrieb erforderliche Funktionalität hat.

Die vorliegende Erfindung schlägt im Hinblick darauf ein Pumpmodul mit einem Ventilblock vor, der zumindest ein Ventil zu dem Zylinder in sich aufnimmt und gegen den Zylinder abgedichtet ist. Das Ventil ist aber üblicherweise in Form einer Ventilbuchse in dem Ventilblock vorgesehen. Bevorzugt weist der Ventilblock zu jedem Zylinder jeweils das Ein- und das Auslassventil, bevorzugt in Form jeweils von Ventilbuchsen mit zugeordneten Ventilkörpern für das Ein- und Auslassventil auf. Darüber hinaus bildet der Ventilblock wenigstens einen zu dem Einlassventil führenden Einlasskanal und wenigstens einen von dem Auslassventil weg führenden Auslasskanal aus. Bevorzugt sind diese Kanäle auf der Oberfläche des Ventilblocks vorgesehen, üblicherweise an einer im Wesentlichen planen Oberfläche ausgeformt, die den Ventilblock an der dem Zylinder
gegenüberliegenden Seite ausbildet. Diese dem Zylinder abgewandte Seite ist mit einem Abdeckelement bedeckt, das an dem Ventilelement anliegt und zwischen sich und dem Ventilblock den Einlasskanal bzw. den Auslasskanal ausbildet. Der Ein- bzw. Auslasskanal ist dabei als zu der Oberfläche des Abdeckelements offenen Seite ausgesparte Nut ausgeformt, die durch das Zusammenwirken von Ventilblock und Abdeckelement zu einem umfänglich geschlossenen Kanal wird, der das mit der Pumpe zu fördernde Fluid zu dem Zylinder führt bzw. von dem Zylinder ableitet.

Durch diese Ausgestaltung ist die Möglichkeit geschaffen, das wesentliche Element einer Pumpe mit Ein- und/oder Auslassventilen auf einfache Weise herzustellen. Dabei wird der Ventilblock üblicherweise mit Ausnehmungen versehen, die sich entweder rechtwinklig zu der Bewegungsrichtung des reversierend beweglichen Pumpkolbens oder parallel hierzu erstrecken. Mit Blick auf eine einfache Herstellung des Ventilblocks ist dieser üblicherweise scheibenförmig ausgebildet. Die ein Ventil aufnehmenden Bohrungen sind dabei üblicherweise sich parallel zu der Bewegungsrichtung des Pumpkolbens erstreckend ausgespart. Dabei sind diese Aussparungen vorzugsweise im Wege des Spritzgießens hergestellt, sodass der Ventilblock ohne Nachbearbeitung die notwendigen Aufnahmen für die Ventile und die Strömungskanäle hat. Es kann notwendig sein, die Spritzgussform mit einem beweglichen Kern zu versehen. Jenseits dessen kann aber die Spritzgussform sehr einfach ausgebildet sein, um die notwendigen Strömungsführungen des Fluids zu dem Zylinder hin und von dem Zylinder weg in den Ventilblock auszuformen.

In ähnlicher Weise ist vorzugsweise das Abdeckelement als Scheibe ausgeformt. Auch hier können an einer oder beiden Hauptseitenflächen der Scheibe Nuten ausgespart sein, die die Strömungskanäle bilden. Üblicherweise ist auch das Abdeckelement als Spritzgussteil in Endkontur hergestellt, d. h. bedarf keiner weiteren Nachbearbeitung. Sämtliche in dem Abdeckelement vorgesehenen Ausnehmungen, die in Form einer Nut oder einer Durchgangsbohrung ausgebildet sein können, erstrecken sich vorzugsweise parallel zu der Bewegungsrichtung des Pumpkolbens.

Mit diesen Erläuterungen ergibt sich, dass die Kombination von Ventilblock und Abdeckelement ein zentrales Element des Pumpmoduls angibt, welches einfach mittels Spritzgießen herzustellen ist und die zu dem oder den Zylindern führenden Strömungskanälen ausbildet und das bzw. die Ventile in sich aufnimmt. Dabei können die Ventile in Form von Ventilbuchsen ausgebildet sein, die einen beweglichen Ventilkörper und eine mit dem Ventilkörper im geschlossenen Zustand der Ventile zusammenwirkende Ventilöffnung ausbilden. Die Ventilbuchsen können aus Kunststoff oder Metall ausgebildet sein und in dem Ventilblock eingepresst sein. Dabei bildet der Ventilblock selbst üblicherweise einen Aufnahmeraum, der in Strömungsrichtung der Ventilöffnung vorgelagert ist und den beweglichen Ventilkörper in sich aufnimmt, sodass dieser sich von seiner geöffneten in seine geschlossene Stellung, bevorzugt allein aufgrund der auf den Ventilkörper wirkenden Druckdifferenz bewegen kann. Der Ventilkörper ist dabei vorzugsweise durch eine frei bewegliche Ventilkugel gebildet, die üblicherweise die Ventilöffnung vollständig verschließen kann.

Der Ventilblock kann einteilig den oder die Zylinder ausbilden. Bei einer solchen Ausgestaltung wird das einteilige Bauteil bevorzugt im Wege des Kunststoff-Spritzgießens und aus Kunststoff hergestellt. Der Ventilblock bildet vorzugsweise aber nicht den Zylinder selbst aus. Vielmehr wird dieser Zylinder üblicherweise als separates Bauteil montiert und dichtend mit dem Ventilblock verbunden. Dementsprechend wird gemäß einer bevorzugten Weiterbildung der vorliegenden Erfindung ein Zylindereinsatz vorgeschlagen, der den Zylinder ausbildet und dichtend gegen den Ventilblock anliegt. Dieser Zylindereinsatz kann aus Kunststoff, insbesondere einem höherwertigen Kunststoff, oder Metall gebildet sein. Der Zylindereinsatz hat dabei an Zylinder zu stellende Oberflächenqualität im Bereich der Innenumfangsfläche, die mit dem Dichtelement des Pumpkolbens dichtend zusammenwirkt. Der Zylindereinsatz kann in einer Gehäusebasis aufgenommen und über diese Gehäusebasis gegen den Ventilblock angelegt, insbesondere dichtend dagegen angepresst sein. Alternativ kann der Zylindereinsatz auch mit dem Ventilblock verpresst sein, derart, dass eine dichte Verbindung zwischen dem Ventileinsatz und dem Ventilblock geschaffen ist. Ebenso gut ist es denkbar, den Zylindereinsatz beim spritzgießtechnischen Herstellen des Ventilblocks zu umspritzen, um damit eine innige Verbindung zwischen dem Ventileinsatz und dem Ventilblock zu schaffen. Auch kann der Zylindereinsatz mit dem Ventilblock verklebt oder verschweißt sein. Dabei ist auf eine fluiddichte Verbindung zwischen dem Ventilblock und dem Zylindereinsatz zu achten.

Für das Verpressen des Zylindereinsatzes mit dem Ventilblock hat dieser üblicherweise einen ringförmigen Vorsprung, der sich über ein gewisses Längenstück des Zylindereinsatzes erstreckt und diesen umfänglich und dichtend umgibt. Zum bestmöglichen Verpressen des Zylindereinsatzes hat dieser üblicherweise als eine Außenumfangsfläche eine Konturierung oder Riffelung, die zusammen mit einer Innenumfangsfläche, die durch den Ventilblock gebildet wird, dichtend zusammenwirkt und den Zylindereinsatz formschlüssig hält.

Dabei ist das Abdeckelement vorzugsweise unmittelbar mit dem Ventilblock verbunden. Diese Verbindung ist vorzugsweise derart, dass das Abdeckelement die in der Phasengrenze zwischen dem Abdeckelement und dem Ventilblock vorgesehenen Ausnehmungen dichtend einsiegelt und so die Ein- bzw. Auslasskanäle ausformt. Die unmittelbare Verbindung ist dabei vorzugsweise durch Verschweißen gebildet. Dementsprechend ist das Abdeckelement bevorzugt aus einem bevorzugt transparenten Kunststoffmaterial ausgebildet, welches für Laser durchlässig ist, wohingegen der Ventilblock aus einem für Laserstrahlen undurchlässigen Kunststoffmaterial gebildet ist. So kann das Abdeckelement von der dem Zylinder gegenüberliegenden Seite mittels Laser-Durchstrahl-Schweißen gegen den Ventilblock geschweißt werden. Dabei werden Laserstrahlen durch das Abdeckmaterial hindurch zu der Phasengrenze geleitet und dort zur Wärme umgeformt. Im Hinblick auf eine gleichmäßige Verschweißung hat es sich als vorteilhaft erwiesen, das Abdeckelement im Wesentlichen als ebene Scheibe auszubilden. Das Abdeckelement hat dementsprechend bevorzugt zwei koplanare Hauptseitenflächen, von denen eine Seitenfläche unmittelbar dichtend an den Ventilblock angelegt und die andere Seite zum Einleiten von Laserstrahlen für das Verschweißen angepasst ausgebildet ist, bevorzugt eben ausgebildet ist. Weitere denkbare Fügeverfahren zum Herstellen der Verbindung sind das UltraschallSchweißen, das Spiegelschweißen, das Kaltverschweißen oder das Verkleben.

Mit Blick auf eine möglichst vereinfachte Herstellung und Montage des Pumpmodules wird gemäß einer bevorzugten Weiterbildung der vorliegenden Erfindung eine vormontierte Pumpeinheit vorgeschlagen, die den zumindest einen Zylindereinsatz, den Ventilblock und das Abdeckelement umfasst. Die Bestandteile dieser Pumpeinheit sind fest miteinander verbunden, sodass die Pumpeinheit im Rahmen der Montage des Pumpmoduls als einheitliches Bauteil gehandhabt werden kann. Diese Pumpeinheit weist regelmäßig auch die Ventilkörper für die Ein- und Auslassventile auf, welche bevorzugt Ventilbuchsen vorgelagert oder in solchen Ventilbuchsen aufgenommen sind. Der Auslass für das in der Pumpeinheit geförderte Fluid wird üblicherweise durch eine in dem Abdeckelement ausgesparte Bohrung gebildet. Der entsprechende Auslass ist bevorzugt an der dem Zylinder gegenüberliegenden Hauptseitenfläche des Abdeckelementes ausgespart. Dieser Auslass kann mit einem stutzenförmigen Auslassanschluss kommunizieren, der mit dem Abdeckelement unmittelbar verbunden ist, beispielsweise daran befestigt oder einteilig daran angeformt ist. Der Auslassanschluss ist aber bevorzugt an einem Kopfelement vorgesehen, welches dem Abdeckelement vorgelagert angeordnet und dicht gegen das Abdeckelement angelegt ist, so dass der an dem Kopfelement vorgesehene Auslassanschluss üblicherweise mit dem Auslass des Abdeckelementes kommuniziert und in axialer Verlängerung hierzu, d. h. bevorzugt in Verlängerung der Bewegungsrichtung des Pumpkolbens sich erstreckend vorgesehen ist. Dementsprechend befindet sich bevorzugt der vorzugsweise stutzenförmige Auslassanschluss an einer Stirnseite des Pumpmoduls. Der Auslassanschluss kann dabei mit einem Gewinde zur Befestigung eines Luer-Anschlusses versehen sein, um einen Druckschlauch an das Pumpmodul anzuschließen.

Dieses Kopfelement ist bevorzugt über zumindest ein Spannelement dichtend regelmäßig unter Zwischenlage eines Dichtelementes, beispielsweise eines Dichtringes angelegt, wobei das Spannelement das Abdeckelement und den Ventilblock durchsetzt. Das Spannelement durchsetzt auch eine gegebenenfalls vorgesehene Gehäusebasis. Sofern ein Kopfelement fehlt, ist das Spannelement anderweitig widergelagert. Bei dem Spannelement handelt es sich vorzugsweise um eine Spannschraube, die sich regelmäßig in Bewegungsrichtung des Pumpkolbens erstreckt. Das gewindeseitige Ende des Spannelementes ist entweder mit dem Kopfelement, oder dem Abdeckelement oder mit einer dem Kopfelement bzw. Abdeckelement vorgelagerten Mutter verbunden. Die Spannschraube kann in dem Kopfelement und/oder dem Abdeckelement im Gewindeeingriff sein.

Die zuvor erwähnte Gehäusebasis ist bevorzugt vorgesehen, um Führungs- und Verriegelungsflächen zur lösbaren Befestigung des Pumpmodules an einem Antriebsgehäuse eines Antriebs auszubilden, dessen Antriebsstößel mit dem Pumpkolben zum reversierenden Betrieb des Pumpkolbens verbindbar ist. So nimmt die bevorzugt vorgesehene Gehäusebasis die Funktion einer Anpassung der Pumpeinheit an dem Antrieb wahr. Die Gehäusebasis kann ferner die Funktion haben, sämtliche funktionalen Elemente der eigentlichen Pumpeinheit zu fassen und in ästhetisch ansprechender Form aufzunehmen bzw. zu umgeben. So hat die Pumpeinheit vorzugsweise einen vorderen Ausgabebereich, der den Ventilblock und/oder das Kopfelement in sich aufnehmen kann. Diese Aufnahme ist vorzugsweise an einer im Wesentlichen zylindrisch ausgeformten Gehäusebasis als stirnseitig offene Ausnehmung ausgebildet. Des Weiteren hat die Gehäusebasis bevorzugt einen hinteren Antriebsbereich, der den Zylinder und/oder den Pumpkolben in sich aufnimmt. Dabei kann der Zylinder bzw. der Pumpkolben ganz oder teilweise in axialer Richtung von der Gehäusebasis axial überdeckt sein.

Die Gehäusebasis kann ferner eine dem Pumpkolben zugeordnete Führungsbuchse aufweisen. Diese Führungsbuchse ist dem eigentlichen Zylinder üblicherweise vorgelagert und dient der Führung des Pumpkolbens beim Pumpbetrieb. Dabei ist die Führungsbuchse üblicherweise nicht derjenige Bereich, in dem beim Pumpbetrieb der Kolben mit seinem Dichtelement dichtend aufgenommen ist und in dem das zu fördernde Fluid komprimiert wird. Vielmehr dient die entsprechende Führungsbuchse bevorzugt allein der Führung des Pumpkolbens in etwa im mittleren Längenbereich desselben.

Das Pumpmodul kann einen oder mehrere Pumpkolben mit zugeordneten Zylindern aufweisen. Bevorzugt sind zumindest zwei Pumpkolben mit zugeordneten Zylindern vorgesehen, die jeweils exzentrisch zu einer Mittellängsachse des länglichen Pumpmoduls vorgesehen sind, sodass durch axiales Verschieben und Drehen nach Art eines Bajonettverschlusses das Pumpmodul an dem Antriebsgehäuse befestigt und dabei gleichzeitig eine formschlüssige Verbindung zwischen den Pumpkolben und den Antriebsstößeln des Antriebs bewirkt werden kann. Jeder Pumpkolben weist im Hinblick darauf bevorzugt ein Formschlusselement auf, welches mit einem Formschlussgegenelement des Antriebsstößels verbindbar ist, um eine reversierende zyklische axiale Bewegung des Antriebsstößels im Wesentlichen spielfrei auf den Pumpkolben zu übertragen.

Gemäß einer weiteren bevorzugten Ausgestaltung der vorliegenden Erfindung wird vorgeschlagen, dass der Einlasskanal mit zumindest zwei Zylindern kommuniziert und der Einlasskanal innerhalb der Phasengrenze zwischen dem Abdeckelement und dem Ventilblock so ausgebildet ist, dass der Einlasskanal den Auslasskanal jedenfalls teilumfänglich umgibt. Danach befindet sich der Auslasskanal innerhalb des Einlasskanals, wobei zu dem Einlassventil führende Kanalabschnitte zwischen sich und einem üblicherweise an der Oberseite des Ventilblocks vorgesehenen Einlassanschluss den Auslasskanal aufnehmen. Diese Ausgestaltung führt bei richtiger Ausrichtung des Pumpmoduls dazu, dass der Einlass jeweils tiefer als der Auslass liegt, wodurch das Einbringen von Luftblasen in die Rückseite des Pumpmoduls in jedem Fall verhindert wird.

Gemäß einer weiteren bevorzugten Ausgestaltung der vorliegenden Erfindung ist an dem Gehäuse ein Transponderelement befestigt. Dieses Transponderelement trägt Informationen zur maximalen Standzeit des Pumpmoduls, d. h. Informationen, die geeignet sind, anzuzeigen, über welche Betriebszeit das Pumpmodul einsetzbar ist. Das Transponderelement kann auch Informationen zum Wirkungsgrad an eine Antriebsvorrichtung weiterleiten, in welche das erfindungsgemäße Pumpmodul eingesetzt wird. Dabei kommuniziert die Antriebseinrichtung mittelbar oder unmittelbar beispielsweise mit einem Handstück, welches als Verbrauchsmaterial zusammen mit einer an die jeweilige Anwendung angepassten Düsengeometrie vertrieben wird, um der Antriebseinrichtung Informationen über den zu erwartenden Betriebspunkt des Düsenquerschnitts zu übermitteln. Durch diese Ausgestaltung ist es möglich, den Betriebspunkt der Antriebseinrichtung an dem Wirkungsgrad des Pumpmoduls zusammen mit dem Handstück und dem darin vorgesehenen Düsenquerschnitt anzupassen. Dabei kann der Transponder eine Spule umfassen, mit welcher das Signal des Handstücks empfangen und in Richtung auf die Antriebseinrichtung verstärkt und weitergeleitet wird. Das Transponderelement enthält ferner Informationen zur Positionierung des Pumpmodules relativ zu dem Antriebsgehäuse. Hierdurch wird sichergestellt, dass der Antrieb aufgrund der gegebenen Lageinformation des Pumpmoduls erst dann in Gang gesetzt wird, wenn eine ordnungsgemäße Befestigung des Pumpmoduls an dem Antriebsgehäuse festgestellt worden ist. Die entsprechenden Informationen des Transponderelementes werden üblicherweise durch eine Leseeinheit ausgelesen, die an dem Antriebsgehäuse vorgesehen ist. Die Leseeinheit kann dabei bevorzugt in Umfangsrichtung eines im Wesentlichen zylindrischen Pumpmoduls an vorbestimmter Stelle vorgesehen sein und erst dann die Lageinformation erhalten und damit auslesen, wenn das Pumpmodul im Rahmen einer Bajonettbewegung durch Drehen in die richtige Position gesetzt worden ist. Das Transponderelement kann auch lediglich der Antriebseinrichtung mitteilen, dass ein Pumpmodul als Verbrauchsteil im Bereich der Antriebseinrichtung vorgesehen ist, wohingegen die richtige Einbaulage des Pumpmoduls relativ zu der Antriebseinrichtung durch einen Schalter angezeigt werden kann, der lediglich dann betätigt wird, wenn das Pumpmodul in richtiger Ausrichtung an dem Antriebsgehäuse festgelegt worden ist. Dabei können beide Maßnahmen miteinander gekoppelt werden, um selbst bei einem eventuell überbrückten Schalter die Antriebseinrichtung lediglich dann betreiben zu können, wenn tatsächlich ein Pumpmodul mit einem Transponderelement in der Nähe der Antriebseinrichtung vorgesehen ist.

Weitere Einzelheiten und Vorteile ergeben sich aus der nachfolgenden Beschreibung eines Ausführungsbeispiels in Verbindung mit der Zeichnung. In dieser zeigen:
- Fig. 1: eine erste Explosionsdarstellung des Ausführungsbeispiels;
- Fig. 2: eine Explosionsdarstellung gemäß Fig. 1 für eine Pumpeinheit des in Fig. 1 gezeigten Ausführungsbeispiels;
- Fig. 3: eine perspektivische Seitenansicht gemäß der Explosionsdarstellung nach Fig. 1 des montierten Pumpmoduls auf den vorderen Ausgabebereich;
- Fig. 4: eine perspektivische Seitenansicht gemäß Fig. 3 in den offenen Antriebsbereich;
- Fig. 5: eine Schnittdarstellung entlang der Linie V-V gemäß Fig. 4 bzw. Fig. 11, wobei die Schnittebene die Mittellängsachse des Pumpenmoduls enthält;
- Fig. 6: eine Schnittansicht entlang der Linie V-V gemäß Fig. 4, wobei die Schnittlinie die Bewegungsebene des Pumpkolbens enthält;
- Fig. 7: das vergrößerte Detail VII gemäß Fig. 6;
- Fig. 8: das vergrößerte Detail VIII gemäß Fig. 6 bei einem gegenüber Fig. 6 tiefer in den Zylinder eindringenden Pumpkolben;
- Fig. 9: eine Schnittdarstellung entlang der Linie IX-IX gemäß Fig. 4, wobei die Schnittebene die Bewegungsebene und die Mittelängsachse eines der Pumpkolben enthält;
- Fig. 10: das vergrößerte Detail X gemäß Fig. 9;
- Fig. 11: eine perspektivische rückseitige Ansicht ähnlich Fig. 4;
- Fig. 12: das vergrößerte Detail XII gemäß Fig. 11;
- Fig. 13: eine perspektivische rückseitige Ansicht der Pumpeinheit nach Fig. 2;
- Fig. 14: eine Schnittdarstellung entlang der Linie XIV-XIV gemäß der Darstellung in Fig. 13;
- Fig. 15: das Detail XV gemäß Fig. 14;
- Fig. 16: eine rückseitige Draufsicht auf das Ausführungsbeispiel;
- Fig. 17: eine Schnittdarstellung entlang der Linie XVII-XVII gemäß der Darstellung nach Fig. 16, wobei die Schnittebene die Mittellängsachsen der beiden Spannschrauben 6 enthält und parallel zu der Bewegungsebene des Pumpkolbens verläuft;
- Fig. 18: eine perspektivische Ansicht eines Ausführungsbeispiels einer Vorrichtung zur Erzeugung eines Fluidstrahles;
- Fig. 19: das Detail nach Figur 18 in vergrößerter Darstellung ohne Pumpenmodul;
- Fig. 20: das Detail nach Figur 18 in einer Draufsicht;
- Fig. 21a-c: eine Ansicht ähnlich zu Fig. 19 mit einer Abfolge von Schritten beim Fügen des Pumpenmoduls und
- Fig. 22a-c: teilweise geschnittene Draufsichten auf die zusammenwirkenden Enden von Antriebselement und Antriebsgegenelement und deren relative Lage beim Schwenken im Rahmen des Fügens; .

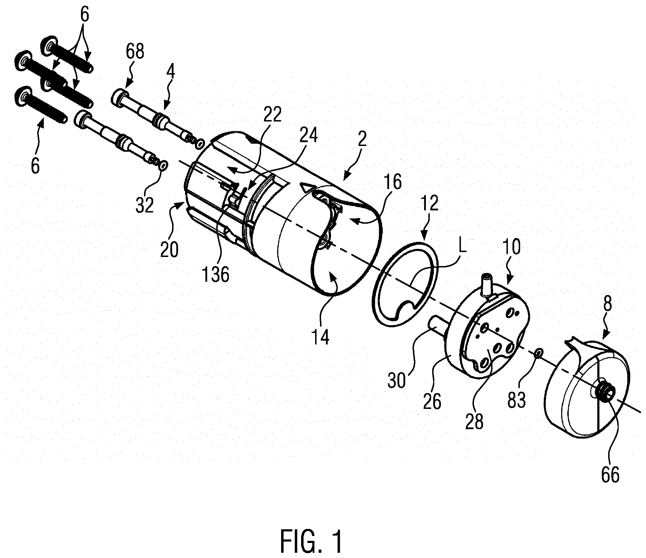

Die Fig. 1 zeigt die wesentlichen Bestandteile des Ausführungsbeispiels nach der vorliegenden Erfindung, bei dem es sich um ein Pumpmodul handelt. Das Pumpmodul hat eine Gehäusebasis 2, die zwei Stößelkörper 4 in sich aufnimmt und reversierend beweglich umgibt. Weiterhin sind vier Spannschrauben 6 dargestellt, die Ausführungsführungsbeispiele von Spannelementen im Sinne der vorliegenden Erfindung sind und im montierten Zustand im Eingriff mit einem Kopfelement 8, welches einer Pumpeinheit 10 vorgelagert ist, die unter Zwischenlage eines ringförmigen RFID-Elementes 12, der ein Beispiel einer Transpondereinheit der vorliegenden Erfindung darstellt, in der Gehäusebasis 2 aufgenommen ist. Hierzu hat die Gehäusebasis 2 einen Ausgabebereich 14, der als zylinderförmige Ausnehmung an der Gehäusebasis 2 ausgebildet ist, wobei ein Axialschlitz 16 zur Aufnahme eines Einlassanschlussstutzens 18 der Pumpeinheit 10 angepasst ausgebildet ist. An dem dem Ausgabebereich 14 gegenüberliegenden Ende ist die Gehäusebasis 2 ebenfalls offen und bildet einen Antriebsbereich 20 aus.

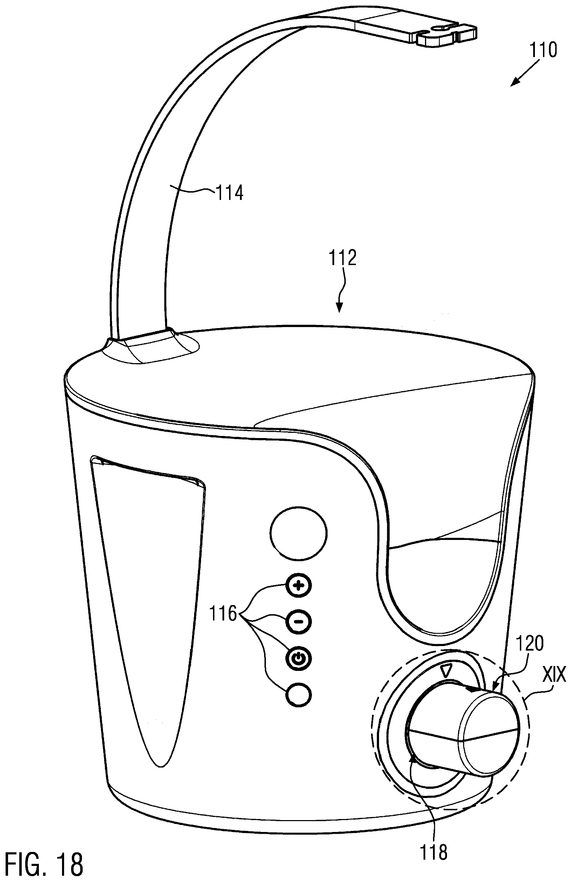

Wie Fig. 1 erkennen lässt, ist die Gehäusebasis im Wesentlichen zylindrisch ausgebildet. An der Außenumfangsfläche sind an der Gehäusebasis 2 eine sich in axialer Richtung der Gehäusebasis 2 erstreckende Nuten 22 und jeweils davon abgehende, sich quer hierzu erstreckende Quernuten 24 ausgebildet, die Führungs- und Verriegelungsflächen zur Befestigung des Pumpmoduls an einem Antriebsgehäuse darstellen, dessen Details in den Fig. 18 ff. und der zugehörigen Beschreibung erläutert sind.

Die Pumpeinheit 10 wird durch einen Ventilblock 26 und ein daran anliegendes Abdeckelement 28 gebildet, wobei von dem Ventilblock 26 auf der dem Abdeckelement 28 gegenüberliegenden Seite zwei Zylindereinsätze 30 abragen, von denen in Fig. 1 lediglich der eine Zylindereinsatz 30 zu erkennen ist, und die im Pumpbetrieb mit den Stößelkörpern 4 zusammenwirken. Die Stößelkörper 4 tragen hierzu jeweils ein Dichtelement 32 in Form eines Dichtringes, der formschlüssig im Bereich des vorderen freien Endes des Stößelkörpers 4 an diesem gehalten ist.

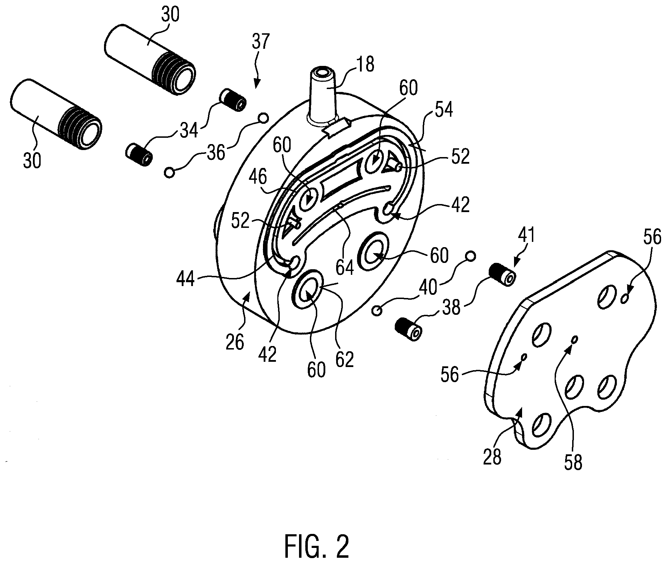

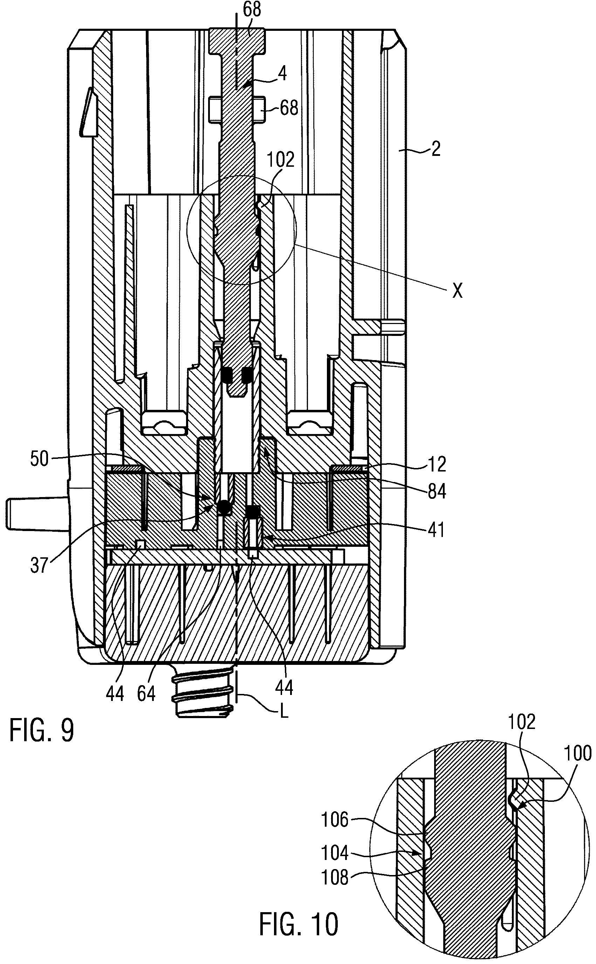

Fig. 2 lässt erkennen, dass die beiden Zylindereinsätze 30 an ihrem dem Ventilblock 26 zugewandten Ende an ihrem Außenumfang eine wellenförmige Konturierung aufweisen, die zum dichtenden Einsetzen der Zylindereinsätze 30 in den Ventilblock 26 ausgeformt sind. Zwischen den Zylindereinsätzen 30 und dem Ventilblock 26 ist jeweils eine Ventilbuchse 34 vorgesehen, die zusammen mit einer Ventilkugel 36 jeweils ein Auslassventil 37 bildet. Auf der den Zylindereinsätzen 30 gegenüberliegenden Seite sind Ventilbuchsen 38 mit zugehörigen Ventilkugeln 40 dargestellt, die zu den jeweiligen Zylindereinsätzen 30 Einlassventile 41 ausformen. Die Einlassventile 41 sind in Einlassventilbohrungen 42 aufgenommen, die in dem Ventilblock 26 ausgespart sind und mit einem Einlasskanal 44 kommunizieren, der in einem Vorsprung 46 als einseitig offene U-förmige Nut ausgespart ist und über das Abdeckelement 28 abgedeckt ist. Die Auslassventile 37 sitzen in entsprechenden Auslassventilbohrungen, von der einer in Fig. 9 beispielhaft gezeigt und mit Bezugszeichen 50 versehen ist. Wie Fig. 2 verdeutlicht, ist der Einlassanschlussstutzen 18 einteilig an dem Ventilblock 26 ausgebildet. Von der im Abdeckelement 28 zugewandten Seite ragen zwei Passelemente 52 mit unterschiedlichem Durchmesser von der durch den Vorsprung 46 gebildeten Dichtfläche 54 ab und überragen diese. Das Abdeckelement 28 hat für diese Passelemente 52 angepasst ausgebildete Bohrungen 56, die der richtigen Positionierung des Abdeckelementes 28 relativ zu dem Ventilblock 26 dienen. Dabei haben die Passelemente 52 und die Passbohrungen 56 einander jeweils angepasste Durchmesser, sodass nach einer Poka-Yoke-Funktion das Abdeckelement 28 immer in der richtigen Ausrichtung und Positionierung bei der Montage des Zylindereinsatzes 30 an dem Ventilblock 26 angeordnet wird.

Neben diesen beiden Passbohrungen 56 weist das Abdeckelement 28 noch eine Auslassbohrung 58 auf.

Der Ventilblock 26 hat korrespondierend zu den Spannschrauben 6 vier Durchgangsbohrungen 60, die einerseits die durch den Vorsprung 46 gebildete Dichtfläche 54 und andererseits Ringflächen 62 durchsetzen, die zur Anlage an das Abdeckelement 28 angepasst ausgebildet und höhengleich vorgesehen sind. An den Flächen 62 und 54 liegt das Abdeckelement 28 dichtend an und ist dagegen mittels Laser-Durchstrahl-Schweißen verschweißt. Hierzu ist das Abdeckelement 28 aus einem Laser-transparenten Material ausgebildet, wohingegen der Ventilblock 46 aus einem Laserstrahlen absorbierenden Kunststoffmaterial ausgeformt ist. Beide Teile können dementsprechend mittels Laser-Durchstrahl-Schweißen verbunden werden, wobei das aus Kunststoff ausgebildete Abdeckelement 28 an der Phasengrenze zu dem Ventilblock 26 mit dem Kunststoffmaterial des Ventilblocks 26 stoffschlüssig verbunden wird. Dadurch werden die Einlasskanäle 44 und ein mit Bezugszeichen 64 gekennzeichnete Auslasskanal, der eine an dem Ventilblock 26 ausgesparte U-förmige Rinne umfasst, die das Abdeckelement 28 abgedeckt ist, gebildet. Der Auslasskanal 64 kommuniziert über die Auslassbohrung 58 mit einer an dem Kopfelement 8 einteilig ausgebildeten Auslassanschlusstülle 66, die in axialer Verlängerung der Auslassbohrung 58 vorgesehen und an ihrem Außenumfang zur Ausbildung eines Luer-Anschlusses mit einem Außengewinde versehen ist. Über eine Überwurfmutter kann dementsprechend auf einfache Weise ein Druckschlauch über eine Luer-Verbindung an die Auslassanschlusstülle 66 angeschlossen werden.

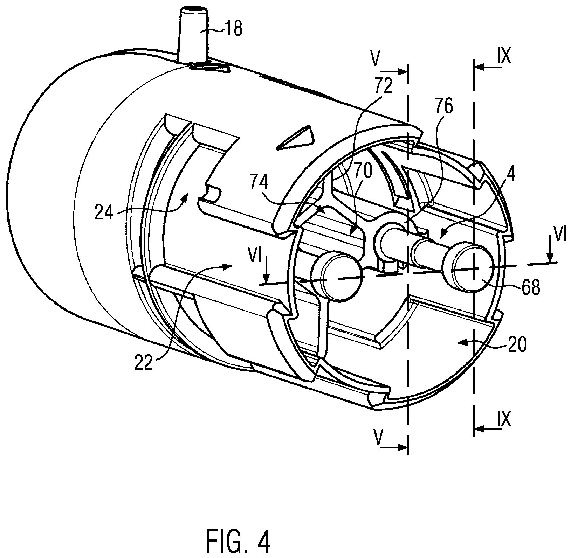

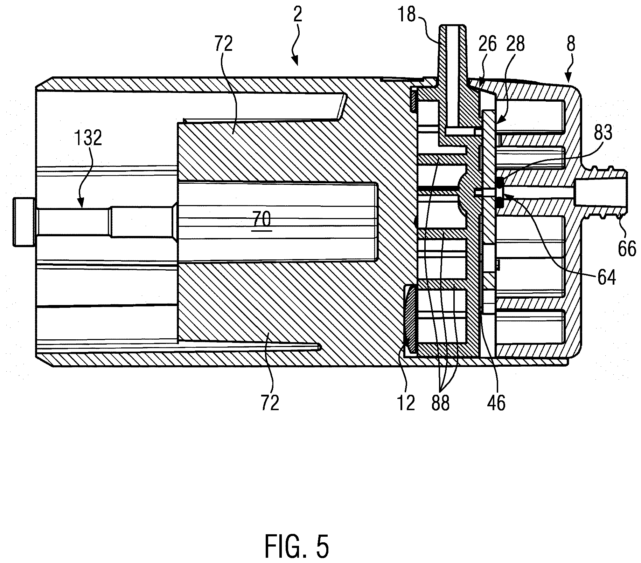

Die Fig. 4 zeigt eine perspektivische Seitenansicht mit einer Draufsicht auf das stirnseitige Ende der Gehäusebasis 2 und des Antriebsbereiches 20. Dabei sind die Stößelkörper 4 von der Gehäusebasis 2 umgeben und ragen mit ihrem einen Ende in den Antriebsbereich 20 hinein. Wie insbesondere Fig. 5 verdeutlicht, überragt das antriebsseitige Ende des Stößelkörpers 4, welches ein als Hammerkopf 68 ausgebildetes Formschlusselement ausbildet, das die Gehäusebasis 2 endseitig überragt. Ansonsten ist der Stößelkörper 4 aber axial von der Gehäusebasis überdeckt (vgl. Figur 5).

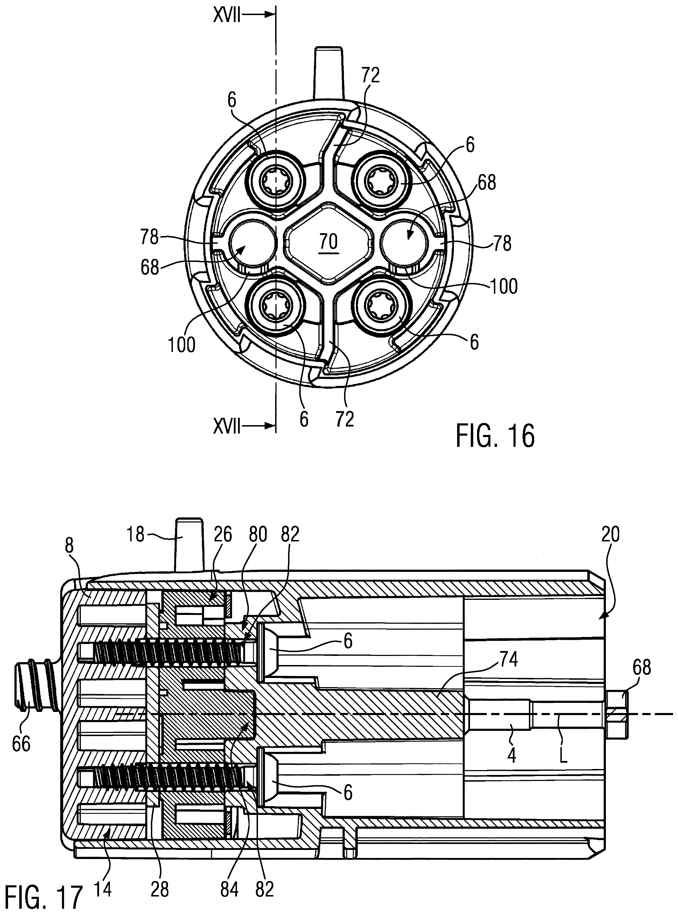

Wie die Zusammenschau der Fig. 4, 5, 6 und 11 verdeutlicht, ist die Gehäusebasis 2 als Spritzgussteil mit relativ gleichmäßigen Wandstärken ausgebildet, sodass sich bei der spritzgießtechnischen Herstellung der Gehäusebasis 2 ein gutes Erstarrungsverhalten ergibt. Als Kunststoffe zur Herstellung der Bauteile des Moduls kommen PA, PE, PP und/oder POM gegebenenfalls als gefüllte Kunststoffe, z. B. gefüllt mit Mineralien und/oder Fasern, in Frage. Dazu hat die Gehäusebasis 2 eine mittlere Ausnehmung 70, die über radiale Stege 72 an die Außenumfangsfläche der Gehäusebasis 2 angebunden ist, wobei die radialen Stege 72 von einer Polygonstruktur 74 abgehen, die zwischen den radialen Stegen 72 Führungsbuchsen 76 zu den jeweiligen Stößelkörpern 4 nach innen anbindet, die über weitere Radialstege 78 an der Außenumfangsfläche der Gehäusebasis 2 abgestützt sind (vgl. Fig. 16).

Die Gehäusebasis 2 bildet eine sich radial erstreckende Trennwand 80 aus, die unter anderem mit Durchtrittsbohrungen 82 für die Spannschrauben 6 versehen ist (vgl. Fig. 17). Dabei durchsetzen die Spannschrauben 6 die Trennwand 80, den Ventilblock 26 und das Abdeckelement 28 vollständig und das Kopfelement 8 teilweise und sind in diesem in Gewindeeingriff. Hierzu sind die Spannschrauben 6 selbstschneidend. Ebenso gut kann das Kopfelement 8 durch Verschweißen mit der durch die Gehäusebasis 2 in dem Ausgabebereich 14 gebildeten Ausnehmung verschweißt und damit mittelbar mit dem Ventilblock 26 und dem Abdeckelement 28 verbunden sein. Ein Dichtring 83 dichtet den durch die Auslassanschlusstülle 66 gebildeten Kanal gegenüber der Auslassbohrung 58 des Abdeckelementes 28 ab (vgl. Fig. 1 und 5).

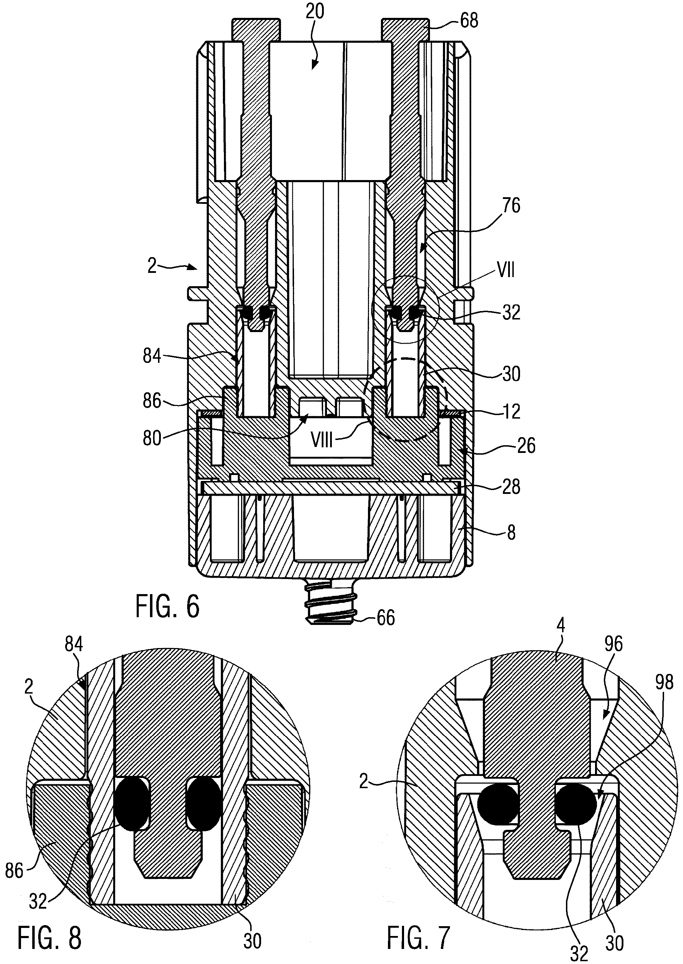

In axialer Verlängerung der Führungsbuchsen 76 bildet die Gehäusebasis 2 bis zu der Trennwand 80 reichende Zylindereinsatz-Aufnahmebohrungen 84 auf, die zur Aufnahme der Zylindereinsätze 30 angepasst ausgebildet sind und in etwa auf Höhe der Trennwand 80 radial verdickt sind, um zwischen dem Zylindereinsatz 30 und dem Material der Gehäusebasis 2 einen Ringraum auszubilden, in welcher ein vorspringender Ringkragen 86 des Ventilblocks 26 passt. Dieser Ringkragen 86 ist beispielsweise in den Fig. 6 und 9 dargestellt. Der Ringkragen 86 dient der dichtenden Verbindung zwischen dem Zylindereinsatz 30 und dem Ventilblock 26. Dabei ist - wie Fig. 8 verdeutlicht - eine konturierte Außenumfangsfläche der Zylindereinsätze 30 innerhalb des Ringkragens 86 aufgenommen und mit diesem auch formschlüssig verrastet. Jeder Zylindereinsatz 30 ist durch Pressen in den Ringkragen 86 eingesetzt und damit dichtend mit dem Ventilblock 26 verbunden.

Die Trennwand 80 bildet ferner eine sich zu dem Ventilblock 26 öffnende Ringnut aus, die zur Aufnahme RFID-Ringes 12 angepasst ausgebildet ist, sodass dieser RFID-Ring 12 zwischen der Trennwand 80 und dem Ventilblock 26 angeordnet werden kann (vgl. Fig. 5). Dabei zeigt Fig. 5 im unteren Teil dieser Ringnut eine Verdickung des RFID-Ringes 12, welcher den Datenträger darstellt. Der übrige, in radialer Richtung verschlankte Bereich des RFID-Ringes 12 dient der angemessenen Positionierung innerhalb der Gehäusebasis 2 (vgl. Fig. 1) und zudem als Spule zur Signalverstärkung eines beispielsweise von einem Handstück abgesetzten Signals, mit dem der Typ der in dem Handstück verbauten Düsengeometrie angezeigt wird.

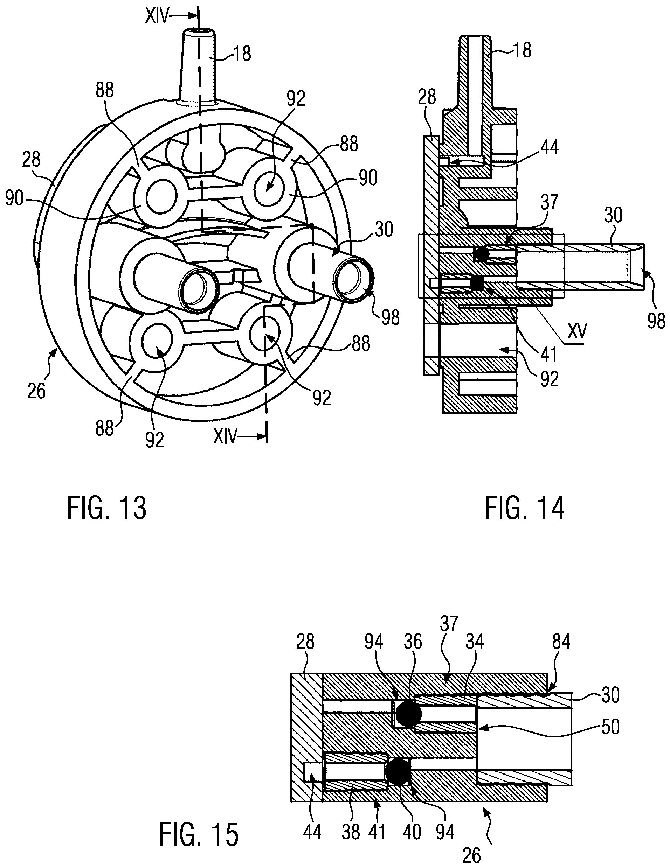

Wie die Fig. 5 und 13 verdeutlichen, ist auch der Ventilblock 26 als Bauteil gleicher Wandstärken ausgebildet und daher gut mittels Kunststoff-Spritzgießen herzustellen. Insbesondere Fig. 5 verdeutlicht mehrere sich in Bewegungsrichtung der Stößelkörper 4 erstreckende Stützrippen 88, die sich an der Trennwand 80 abstützen und Hülsensegmente 90 anbinden, die Durchtrittsbohrungen 92 für die Spannschrauben 6 ausformen, die mit den Durchtrittsbohrungen 82 durch die Trennwand 80 fluchten, wobei die Hülsensegmente 90 die zuvor erwähnten Ringflächen 62 zur Anlage des Abdeckelementes 28 ausbilden.

Die Fig. 14 und 15 verdeutlichen die Anordnungen der Ein- und Auslassventile 37, 41 in dem Ventilblock 26. Dieser Ventilblock 26 hat zur Aufnahme der entsprechenden Ventilbuchsen 34 und 38 angepasste Bohrungen 42, 50, denen jeweils in Strömungsrichtung des Fluids ein Aufnahmeraum 94 nachgelagert zugeordnet ist, in dem sich die Ventilkugel 36 bzw. 40 befindet. Diese Ventilkugel 36 bzw. 40 wirkt im geschlossenen Zustand des Ventils mit einer Ventilöffnung zusammen, die durch das strömungsferne Ende der entsprechenden Ventilbuchse 34, 38 gebildet ist. In Fig. 15 ist diese Position für die Ventilkugel 36 des Auslassventiles 37 gezeigt, wohingegen die Ventilkugel 40 des Einlassventils 41 die entsprechende Ventilöffnung freigibt. So verdeutlicht Fig. 15 einen Zustand, bei dem der Stößelkörper 4 den Hubraum innerhalb des Zylindereinsatzes 30 vergrößert und zu pumpende Flüssigkeit durch den Einlasskanal 44 in den Hubraum eingeleitet wird, wohingegen der Auslasskanal durch das Auslassventil 37 verschlossen ist. Die jeweiligen Ventilkugeln 36, 40 sind bei dem gezeigten Ausführungsbeispiel frei beweglich in dem Aufnahmeraum 94 vorgesehen und allein aufgrund der Durchmesserverhältnisse zwischen der Ventilöffnung und dem strömungsfernen Durchmesser des von der Ventilöffnung abgehenden und in dem Ventilblock 26 ausgebildeten Kanals in dem Ventilblock 26 unverlierbar gehalten. Zur Montage wird zunächst die entsprechende Kugel 36, 40 in den Aufnahmeraum 94 eingesetzt. Danach wird die Ventilbuchse 34 bzw. 38 in den Ventilblock 26 eingepresst. Danach sind die Ventile 37, 41 unverlierbar in dem Ventilblock 26 vormontiert.

Wie Fig. 15 ferner erkennen lässt, liegt der in den Ventilblock 26 eingepresste Zylindereinsatz 30 stirnseitig gegen die Ventilbuchse 34 des Auslassventiles 37 an, wodurch das auf der Druckseite der Pumpe vorgesehene Ventil 37 zusätzlich lagegesichert und gegen unerwünschtes Herauspressen aus der Presspassung zu der Ventilbuchse 36 gehindert wird.

Insbesondere die Fig. 7 verdeutlicht einen ersten konischen Einzug 96, der durch die Gehäusebasis 2 ausgebildet ist und den Zylindereinsatz 30 in Richtung auf den Antriebsbereich 20 vorgelagert vorgesehen. Dieser erste konische Einzug 96 erleichtert das Einbringen des Stößelkörpers 4 mit seinem vorderen Ende, an dem sich das Dichtelement 32 befindet, in den durch den Zylindereinsatz 30 gebildeten Zylinder. Beim Einbringen des Stößelkörpers 4 wird das Dichtelement 32 konzentrisch zu dem Zylindereinsatz 30 angeordnet und in etwa auf dessen Innendurchmesser gebracht. Ein zweiter konischer Einzug 98 wird durch den Zylindereinsatz 30 selbst ausgebildet. Innerhalb dieses zweiten konischen Einzugs 98 befindet sich das Dichtelement 32 in der in den Fig. 6 und 7 verdeutlichten Parkposition. So ist das Dichtelement 32 mit radialem Abstand zu dem Zylindereinsatz 30 vorgesehen. Der sich dadurch bildende Radialspalt erlaubt einen Durchtritt von Flüssigkeit und/oder Gas für die Sterilisation bzw. Desinfektion des Ausführungsbeispiels nach der Montage sämtlicher Bauteile. Diese Parkposition wird durch ein Sicherungselement festgelegt, welches vorliegend durch eine einteilig an der Gehäusebasis 2 angeformte Rastklinke 100 ausgebildet ist. Diese Rastklinke 100 ist insbesondere den Fig. 10 bis 12 zu entnehmen. Die Rastklinke 100 ist durch Freischneiden des antriebsseitigen Endes der Führungsbuchse 76 ausgebildet. Die Rastklinke 100 hat einen Verriegelungsvorsprung 102, der in den Fig. 9 und 10 verdeutlicht ist und in der Parkposition in eine Verriegelungsnut 104 eingreift, die zwischen zwei einteilig an dem Stößelkörper 4 einteilig ausgebildete ringförmige Vorsprünge 106, 108 ausgebildet ist (vgl. Fig. 10). Dabei bildet der vordere ringförmige Vorsprung 108 eine sich nahezu streng radial erstreckende Flanke der Verriegelungsnut 104 aus, wohingegen der hintere ringförmige Vorsprung 106 eine schräge Flanke hat, die das Vorschieben des Stößelkörpers 4 von der Parkposition in eine Pump- bzw. Betriebsposition, erleichtert. In einer Pump- bzw. Betriebsposition befindet sich das Dichtelement 32 in dichtender Anlage an der Innenumfangsfläche des Zylinders, vorliegend des Zylinderelementes 30. Dabei kann davon ausgegangen werden, dass die Fig. 9 und 10 die oberste Pumpposition und Fig. 8 die unterste Pumpposition wiedergeben. Zwischen diesen beiden Positionen nach den Fig. 8 und 9 findet der Hub des Stößelkörpers 4 statt.

Durch die Ausgestaltung von Rastklinke 100 und Verriegelungsnut 104 wird die zuvor beschriebene Parkposition gesichert. Axialer Druck gegen den Stößelkörper 4 von der Antriebsseite führt jenseits eines kritischen Wertes der Druckkraft dazu, dass die Parkposition aufgehoben und der Stößelkörper 4 tiefer in das Gehäuse und in die Pumpposition verschoben wird. In dieser Pumpposition führen die Vorsprünge 106, 108 den Stößelkörper 4 auch gegenüber der Führungsbuchse 76, die durch die Gehäusebasis 2 ausgebildet wird (vgl. Fig. 9, 10), wodurch eine höhere Laufruhe des Stößelkörpers 4 beim Pumpbetrieb erreicht wird. Insbesondere wird ein Knicken des Stößelkörpers 4 bei axialer Beanspruchung verhindert, sodass der Stößelkörper 4 aus einem relativ weichen Material, wie beispielsweise Kunststoff, hergestellt werden kann.

Wie Fig. 6 verdeutlicht, überragt der Stößelkörper 4 in der Parkposition mit seinem Hammerkopf 68 die Gehäusebasis 2, wodurch ein optischer Indikator zur Überprüfung der Parkposition gegeben ist. Nach dem Verbinden mit dem Antrieb, bei welchem zwangsläufig die Stößelkörper 4 von der Parkposition in eine Pumpposition überführt werden, liegen die antriebsseitigen Enden mit dem Hammerkopf 68 jeweils innerhalb der Gehäusebasis 2 und der durch die dort im Antriebsbereich 20 gebildete axial offene hintere Ausnehmung frei.

Wie die Beschreibung des Ausführungsbeispiels verdeutlicht, sind bei dem erfindungsgemäßen Pumpmodul zwischen dem Zylindereinsatz 30 und dem Dichtelement 32 die Ein- und Auslasskanäle 44, 64 ausgebildet. Diese erstrecken sich innerhalb einer Phasengrenze zwischen dem Ventilblock 26 und dem Abdeckelement 28. Der hier vorgesehene Einlasskanal 44 verteilt eingeleitete Flüssigkeit von einem oberen Ende nahe des Einlassanschlussstutzens 18 zu den jeweiligen Einlassventilen 41. Das Fluid wird in der Phasengrenze bis zu den Einlassventilen 41 am äußeren Rand der Phasengrenze geführt und umgibt dementsprechend zumindest teilweise den Auslasskanal 64. Dieser Auslasskanal 64 kommuniziert mit mehreren, vorliegend zwei, Auslassventilen 37. Innerhalb der Phasengrenze zwischen dem Abdeckelement 28 und dem Ventilblock 26 leitet der Auslasskanal 64 das unter Druck befindliche Fluid bis hin zu einem Sammelpunkt, der mit dem durch die Auslassanschlusstülle 66 gebildeten Abgabekanal fluchtet. Der Sammelpunkt befindet sich dabei ebenfalls innerhalb der Phasengrenze zwischen dem Abdeckelement 28 und dem Ventilblock 26. Insbesondere wird der überwiegende Teil von Einlasskanal 44und/oder Auslasskanal 64 innerhalb der Phasengrenze zwischen dem Ventilblock 26 und dem Abdeckelement 28 gebildet. Der überwiegende Teil stellt dabei zumindest 50 %, bevorzugt 60 % der gesamten Länge des Strömungsweges des entsprechenden Kanals innerhalb des Pumpmodules dar. Dieser Strömungsweg beginnt für die Einlassseite mit der Einlassöffnung des Einlassstutzens 18 und endet an dem Einlassventil 41. Der entsprechende Weg beginnt auslassseitig mit der durch die Auslassanschlusstülle 66 gebildete Öffnung und endet an dem Auslassventil 37, vorliegend dem Aufnahmeraum 94 des entsprechenden Ventils 37.

Bedeutsam für die Erfindung ist ferner die Pumpeinheit 10, die aus dem Ventilblock 26 und dem Abdeckelement 28 mit den darin eingebauten Ventilen 37, 41 und den Zylindereinsätzen 30 besteht. Diese Pumpeinheit 10 ist vormontiert. Dabei kann die Erfindung auch insofern variiert werden, indem der Zylinder durch die Gehäusebasis 2 selbst oder ein in die Gehäusebasis 2 aufgenommenes Zylinderelement gebildet wird, welches dichtend gegen den Ventilblock 26 angelegt wird. So ist vorstellbar, dass der in Fig. 7 zu erkennende Kragen im Anschluss an den ersten konischen Einzug 96 unmittelbar an einem Zylindereinsatz anliegt und diesen durch Vorspannung der Gehäusebasis 2 unter Vorspannung gegen den Ventilblock 26 andrückt, und zwar zusammen mit einem O-Ring, der an der Phasengrenze zwischen der Gehäusebasis 2 und dem Ventilblock 26 angeordnet werden kann und den so vorgesehenen Zylindereinsatz abdichtet.

Weiterhin ist bedeutsam, dass eine Parkposition definiert ist, in welcher der durch die Stößelkörper 4 gebildete Pumpkolben festgelegt ist, derart, dass bei gewissem axialem Druck der Stößelkörper 4 aus der Parkposition heraus in eine Pumpposition verschoben wird. In der Parkposition liegt jedenfalls das Dichtelement 32 nicht an der Innenumfangsfläche des zugeordneten Pumpzylinders an. Das Dichtelement 32 ist regelmäßig mit radialem Abstand zu benachbarten Gehäuseteilen des Pumpmodules vorgesehen, sodass die Sterilisation bzw. Desinfektion an dem Zylinder und dem Kolben vorbei erfolgen kann. Sämtliche strömungsführenden Teile des Pumpmodules werden hierbei vollständig mit dem Desinfektions- bzw. Sterilisationsmittel überstrichen und damit wirkungsvoll sterilisiert.

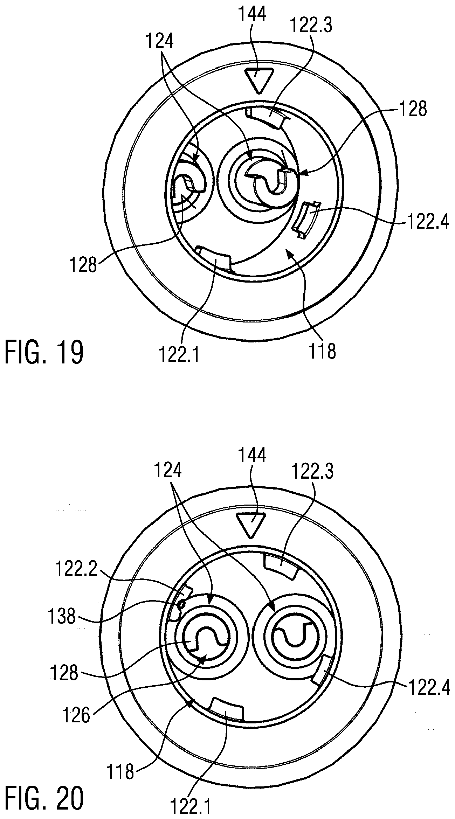

Die Figur 19 zeigt eine perspektivische Seitenansicht eines Ausführungsbeispiels einer Antriebseinheit 110 mit einem in einem Antriebsgehäuse 112 vorgesehenen Antrieb, bei dem es sich um einen elektrischen Antrieb handelt. Von dem Antriebsgehäuse 112 ragt eine Halterung 114 zur Halterung eines Flüssigkeitsbeutels ab. An dem Antriebsgehäuse 112 liegen ferner verschiedene Bedienelemente 116 frei, die der Steuerung des Antriebs sowie dem Ein- bzw. Ausschalten des Antriebs dienen. Bezugszeichen 118 kennzeichnet eine im Wesentlichen zylindrische Ausnehmung, in die ein mit Bezugszeichen 120 gekennzeichnetes Pumpmodul nach den Figuren 1 bis 17 eingesetzt ist, welches gegenüber diesen Figuren vereinfacht dargestellt ist. Die Gehäusebasis 2 hat nach innen in die Ausnehmung 118 vorspringende Nocken 122, die Ausführungsbeispiele für Formschlusselemente der vorliegenden Erfindung sind. Es sind vorliegend vier Nocken 122 auf dem Umfang verteilt vorgesehen. Die mit Bezugszeichen 122.4 gekennzeichnete Nocke kann eine geringere radiale Erstreckung und eine geringere Erstreckung in Umfangsrichtung als die übrigen Nocken 122.1 bis 122.3 haben, um eine eindeutige Zuordnung des Pumpmoduls 120 zu erlauben. Andere Arten einer Poka-yoke-Gestaltung sind denkbar. So können Nuten mit unterschiedlichem Winkelversatz relativ zueinander auf der Außenumfangsfläche des Gehäuses, namentlich der Gehäusebasis 2, vorgesehen sein, so dass das Pumpmodul 120 lediglich in einer vorbestimmten Weise in die Ausnehmung 118 eingesetzt werden kann. In der Ausnehmung 118 liegen ferner Antriebselemente in Form von Antriebsstößeln 124 frei, die mit dem innerhalb des Antriebsgehäuses 112 vorgesehenen Antrieb verbunden und in Längsrichtung reversierend angetrieben hin und her beweglich sind. Die Antriebsstößel 124 bilden eine Anschlagfläche 126 aus. Vorliegend sind zwei Antriebsstößel 124 vorgesehen. Die Anschlagfläche 126 wird durch eine in der Draufsicht C-förmige Klaue 128 überragt, die zwischen sich und der Anschlagfläche 126 eine Hammerkopfaufnahme 130 ausbildet.

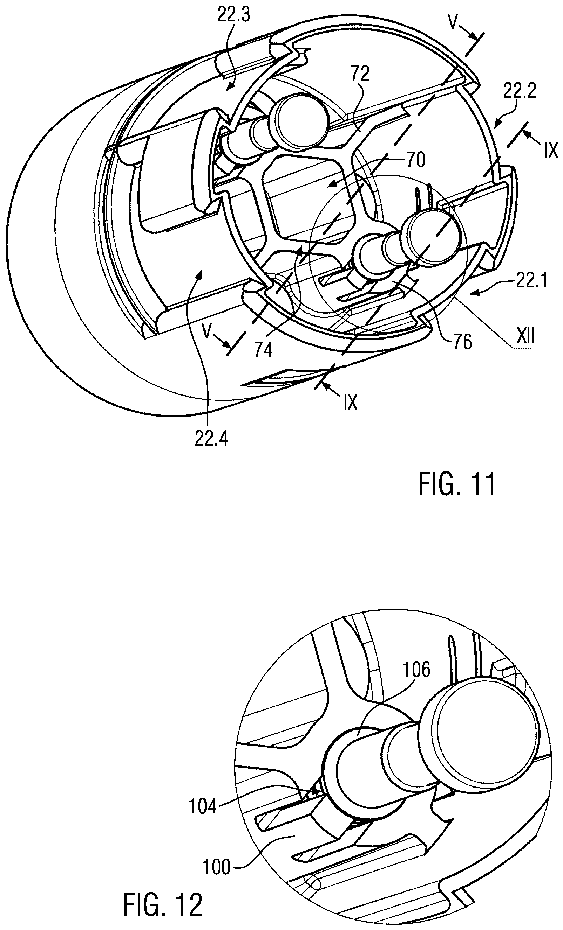

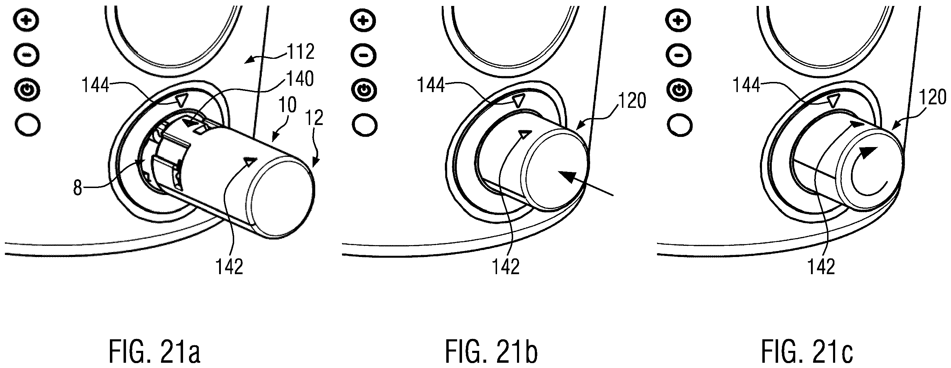

Wie insbesondere die Figuren 11 und 16 erkennen lassen, ist von den vier Nuten 22 am Außenumfang der Gehäusebasis 2, die sich streng in axialer Richtung entlang der Mittellängsachse L erstrecken, die mit Bezugszeichen 22.4 gekennzeichnete Nut zur exakten Aufnahme der kleineren Nocke 122.4 angepasst ausgebildet. Durch das Zusammenwirken insbesondere der kleineren Nocke 122.4 mit der kleineren Nut 22.4 wird eine eineindeutige Ausrichtung des Pumpmoduls 120 beim Fügen, d.h. beim Einschieben des Pumpmoduls 120 in die Ausnehmung 116 vorgegeben. Damit kann das Pumpmodul 120 nur in einem Winkel rechtwinklig zu einer in Figur 21c gezeigten Endlage um 30° versetzt eingeführt werden. Diese geschwenkte Lage ist in Figur 21b verdeutlicht. Der Hammerkopf 68 überragt einen endseitigen Pumpkolbenabschnitt 132 jedes einzelnen Pumpkolbens 4, der einen geringeren Durchmesser als der übrige Pumpkolben 4 hat. Der Hammerkopf 68 definiert das stirnseitige, anschlussseitige Ende des Pumpkolbens 4 und bildet hier eine Gegenfläche 134 zu der Anschlagfläche 126 aus.

Die Nut 22 bildet zusammen mit der Quernut 24 eine Führung eines Bajonettverschlusses mit dem jeweiligen Nocken 122, um zunächst eine axiale Einführbewegung zu führen, die ihr Ende dann findet, wenn die Nocken 122 gegen das innenseitige untere Ende der Nuten 22 anstoßen, um danach in einer Schwenkbewegung in die Quernut 24 schwenkt und damit axial arretiert zu werden. In der endseitig gegen die Quernut 24 anliegenden Endlage kann ein Rastvorsprung wirksam sein, der eine Verdrehsicherung zwischen dem Pumpmodul 112 und dem Antriebsgehäuse 2 ausbildet, sodass das Pumpmodul 112 in seiner Endlage gesichert ist.

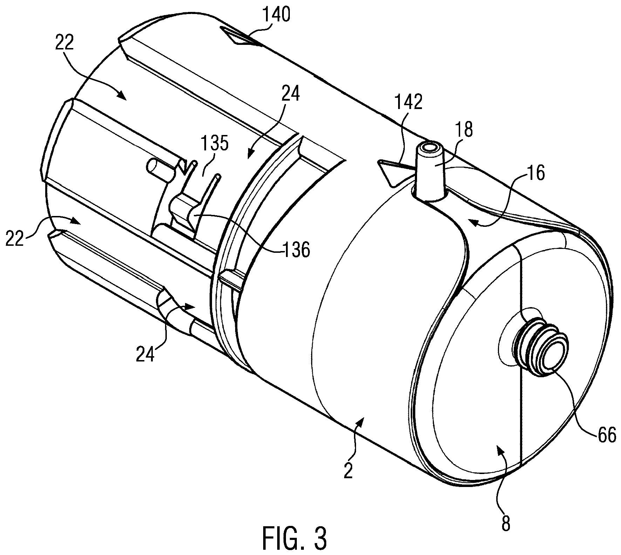

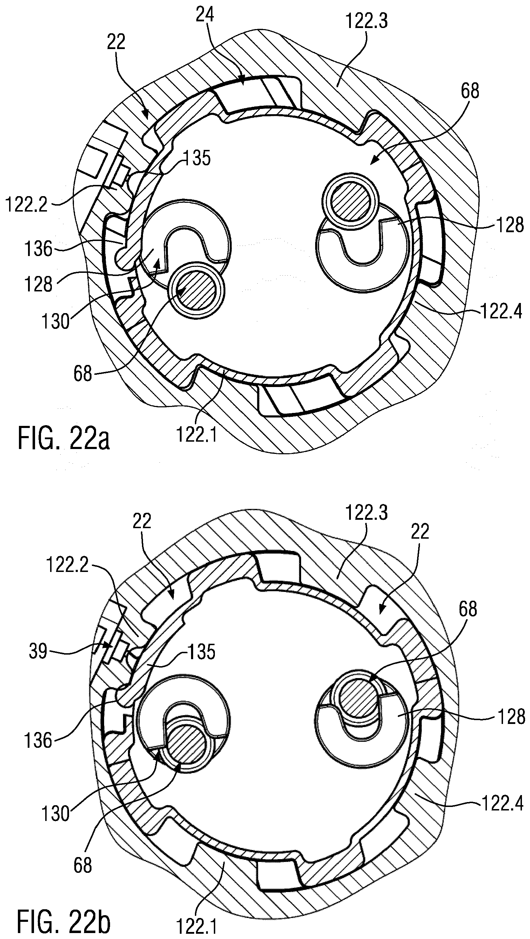

In Fig. 22a ist ferner innerhalb der Quernut 24 ein an einem Federarm 135 ausgebildeter Rast- und Schaltvorsprung 136 eingezeichnet, der in der Quernut 24 freiliegt und fest an der Gehäusebasis 2 ausgebildet ist (vgl. Fig. 3). Diesem Rast- und Schaltvorsprung 136 ist ein mittig in der Nocke 122.2 vorgesehener Schalter 138 zugeordnet. Der Schalter 138 ist in radialer Richtung nach innen in Bezug auf die Ausnehmung 118 vorgespannt und wirkt dementsprechend mit dem Rast- und Schaltvorsprung 136 zusammen. Durch Betätigung dieses Schalters 138 durch den Rast- und Schaltvorsprung 136 wird erst die Möglichkeit gegeben, die Antriebsstößel 124 anzutreiben. Ist dementsprechend das Pumpmodul 10 nicht in der vorgeschriebenen Weise mit der Antriebseinheit 1 verbunden, kann die Antriebseinheit nicht betrieben werden. Zusätzlich ist das Antriebsgehäuse 112 mit einer Leseeinheit versehen, welche die richtige Ausrichtung des RFID-Ringes 12 und damit des Pumpmoduls 120 relativ zu dem Antriebsgehäuse 112 erkennt und erst dann den Abtrieb freigibt. Damit wird ein Betrieb der Vorrichtung bei überbrücktem Schalter 138 verhindert.

Die Figuren 21a bis c zeigen das Einführen des Pumpmoduls 120 in die Ausnehmung 118. Wie bereits zuvor erwähnt, wird das Pumpmodul 120 um 30° im Gegenuhrzeigersinn relativ zu der Endlage zunächst geschwenkt, um die Nocken 122 mit den Nuten 22 zur Überdeckung zu bringen (vgl. Figur 21a). Die geschwenkte Position wird durch einen Ausrichtungspfeil 140 gekennzeichnet, der in Figur 3 deutlich zu erkennen ist und in Figur 21a mit einer gehäuseseitig vorgesehenen Gegenmarkierung 144 fluchtet. In dieser relativen Ausrichtung kann das Pumpmodul 120 nunmehr in die Ausnehmung 118 eingeschoben werden. Diese axiale Einschiebebewegung wird geführt durch die Nocken 122, die in die korrespondierend hierzu ausgebildeten Nuten 22 eingreifen. In der Darstellung nach Figur 21b ist dieses axiale Einschieben, das in Figur 21b mit einem gradlinigen Pfeil verdeutlicht wird, beendet. Das Pumpmodul 120 ist nunmehr maximal in die Ausnehmung 118 eingeschoben. Danach wird das Pumpmodul 120 um 30° im Uhrzeigersinn geschwenkt, wie dies der Pfeil in Figur 21c andeutet. Nach dieser Schwenkbewegung um 30° hat das Pumpmodul 120 seine Endposition erreicht. Die Endposition wird dem Benutzer auch durch einen auf dem Außenumfang der Gehäusebasis 2 vorgesehenen Richtungspfeil 142 angeben, der in der Endposition mit der Gegenmarkierung 144, die an dem Antriebsgehäuse 2 vorgesehen ist, fluchtet. Der Richtungspfeil 142 gibt auch die Richtung des Einführens für das Pumpmodul 120 in die Ausnehmung 118 vor.

Beim Fügen von Pumpmodul 120 und Antriebsgehäuse 112 werden die Antriebsstößel 124 und die Pumpkolben 4 einander angenähert. Aufgrund der axialen Führung der Nocken 122 in den Nuten 22 befindet sich die durch den Hammerkopf 68 gebildete Gegenfläche 134 zumindest teilweise über der durch den Antriebsstößel 124 gebildeten Anschlagfläche 126 (vgl. Figur 22a). So führt eine fortschreitende axiale Bewegung schließlich dazu, dass der Pumpkolben 4 endseitig gegen die Anschlagfläche 126 angelegt wird. Mit weiter zunehmender Annäherung des Pumpmoduls 120 an das Antriebsgehäuse 112 wird die Parkposition aufgehoben und der Pumpkolben 4 tiefer in die Gehäusebasis 2 und in eine Pumpposition gedrängt. Es ergibt sich danach keine weitere relative axiale Bewegung zwischen dem Antriebsstößel 124 und dem zugeordneten Pumpkolben 4.

Der jeweilige Hammerkopf 68 der beiden Pumpkolben 4 befindet sich dabei in einer exzentrischen Position relativ zu dem Mittelpunkt des Antriebsstößels 124, die in Figur 22a gezeigt ist. Üblicherweise wird nach dem axialen Anlegen beider Pumpkolben 4 an die Antriebsstößel 124 die Gehäusebasis 2 um einen weiteren, geringfügigen Weg axial relativ zu dem Antriebsgehäuse 2 verschoben, sodass sichergestellt wird, dass bis zum Erreichen der axialen Endlage beim Fügen von Pumpmodul 120 und Antriebsgehäuse 112 jederzeit zuverlässig eine axiale Anlage des Pumpenkolbens 4 an dem Antriebsstößel 124 erreicht wird, bevor die Gehäusebasis 2 relativ zu dem Antriebsgehäuse 112 geschwenkt wird. Jedenfalls sollte die Ausgestaltung so sein, dass bei jeder erdenklichen Positionierung des Antriebsstößels 124, selbst bei einer Positionierung des Antriebsstößels 124 in der tiefsten Lage innerhalb der Ausnehmung 8 eine sichere Anlage des Pumpkolbens 4 gegen den Antriebsstößel 124 nach Beendigung der axialen Einschiebebewegung erreicht ist.

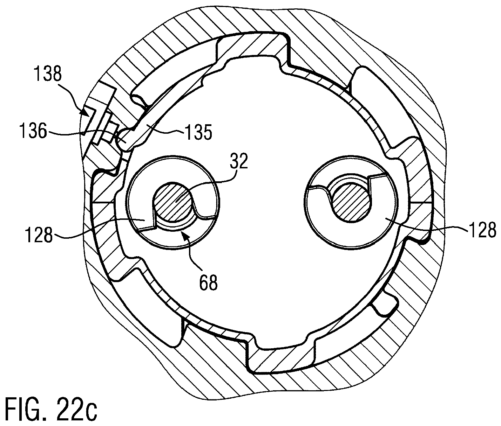

Nach Erreichen dieser axialen Endlage wird das Pumpmodul 120 nunmehr im Uhrzeigersinn geschwenkt. Dadurch werden - wie die Figuren 22a bis 22c verdeutlichen - die exzentrisch zu dem Mittelpunkt dieser Schwenkbewegung angeordneten Hammerköpfe 68 mit ihrer Gegenfläche 134 gleitend auf der Anschlagfläche 126 relativ zu dem Antriebsstößel 124 verschoben, und zwar in einer Ebene, die sich rechtwinklig zu der Einschieberichtung erstreckt. Die zuvor exzentrische Anordnung der Pumpkolben 4 relativ zu den Antriebsstößeln 124 gemäß Figur 22a nähert sich danach über eine in Figur 22b gezeigte Zwischenposition der in Figur 22c gezeigten Endposition an. In dieser Endposition stoßen die Nocken 122 gegen Anschläge an, die durch die Quernuten 24 gebildet sind. Die Gehäusebasis 2 wird üblicherweise gegen das Antriebsgehäuse 2 verriegelt. Die Pumpkolben 4 sind im Wesentlichen konzentrisch zu den Antriebsstößeln 124 angeordnet. Jede Klaue 128 übergreift den zugeordneten Hammerkopf 68. So ist der Hammerkopf 68 durch Übergriff der die Klaue 128 aufweisenden Hammerkopfaufnahme 130 axial formschlüssig gehalten. Üblicherweise ist die Hammerkopfaufnahme 130 in axialer Richtung exakt auf die Höhe des Hammerkopfes 68 abgestimmt, sodass sich eine spielfreie axiale formschlüssige Verbindung zwischen dem Antriebsstößel 124 und dem Pumpkolben 4 ergibt.

### Bezugszeichenliste

- 2: Gehäusebasis
- 4: Stößelkörper/Pumpkolben
- 6: Spannschraube
- 8: Kopfelement
- 10: Pumpeinheit
- 12: RFID-Ring
- 14: Ausgabebereich
- 16: Axialschlitz
- 18: Einlassanschlussstutzen
- 20: Antriebsbereich
- 22: Nut
- 24: Quernut
- 26: Ventilblock
- 28: Abdeckelement
- 30: Zylindereinsatz
- 32: Dichtelement
- 34: Ventilbuchse
- 36: Ventilkugel
- 37: Auslassventil
- 38: Ventilbuchse
- 40: Ventilkugel
- 41: Einlassventil
- 42: Einlassventilbohrung
- 44: Einlasskanal
- 46: Vorsprung
- 50: Auslassventilbohrung
- 52: Passelement
- 54: Dichtfläche
- 56: Passbohrung
- 58: Auslassbohrung
- 60: Durchgangsbohrungen
- 62: Ringfläche
- 64: Auslasskanal
- 66: Auslassanschlusstülle
- 68: Hammerkopf
- 70: mittlere Ausnehmung
- 72: radialer Steg
- 74: Polygonstruktur
- 76: Führungsbuchse
- 78: weiterer radialer Steg
- 80: Trennwand
- 82: Durchtrittsbohrung
- 83: Dichtring
- 84: Zylindereinsatz-Aufnahmebohrung
- 86: Ringkragen
- 88: Stützrippe
- 90: Hülsensegment
- 92: Durchtrittsbohrung
- 94: Aufnahmeraum
- 96: erster konischer Einzug
- 98: zweiter konischer Einzug
- 100: Rastklinke
- 102: Verriegelungsvorsprung
- 104: Verriegelungsnut
- 106: ringförmiger Vorsprung
- 108: ringförmiger Vorsprung
- 110: Antriebseinheit
- 112: Antriebsgehäuse
- 114: Halterung
- 116: Bedienelement
- 118: Ausnehmung
- 120: Pumpmodul
- 122: Nocken
- 124: Antriebsstößel
- 126: Anschlagfläche
- 128: Klaue
- 130: Hammerkopfaufnahme
- 132: Pumpkolbenabschnitt
- 134: Gegenfläche
- 135: Federarm
- 136: Rast- und Schaltvorsprung
- 138: Schalter
- 140: Ausrichtungspfeil
- 142: Richtungspfeil
- 144: Gegenmarkierung
- L: Mittellängsachse

## Patentansprüche

1. Pumpmodul mit einem Pumpengehäuse (2, 8, 26, 28), einem Zylinder (30), zumindest einem Pumpkolben (4), der in dem Pumpengehäuse reversierend beweglich gelagert ist und der mit zumindest einem Dichtelement (32) versehen ist, das in einem Pumpbetrieb mit dem Zylinder (30) zusammenwirkt, einem gegen den Zylinder (30) abgedichteten Ventilblock (26), zumindest einem Ventil (37, 41) zu dem Zylinder, das in dem Ventilblock (26) aufgenommen ist, und einem Abdeckelement (28), das auf einer dem Zylinder gegenüberliegenden Seite an dem Ventilblock (26) anliegt und zwischen sich und dem Ventilblock (26) einen zu dem Zylinder (30) führenden Einlasskanal (44) und einen mit dem Zylinder (30) kommunizierenden Auslasskanal (64) ausbildet, **dadurch gekennzeichnet,**
**dass** der Einlass- (44) und der Auslasskanal (64) jeweils durch eine zu der Oberfläche des Abdeckelements (28) offene Nut in der dem Zylinder (30) gegenüberliegenden Seite des Ventilblocks (26) gebildet und durch das Zusammenwirken von Ventilblock (26) und Abdeckelement (28) umfänglich geschlossen ist und sich rechtwinklig zu der Bewegungsrichtung des Pumpkolbens erstreckt.

2. Pumpmodul nach Anspruch 1, **dadurch gekennzeichnet dass** der Ventilblock (26) einen Anschluss (18) für in der Pumpe zu förderndes Fluid aufweist.

3. Pumpmodul nach Anspruch 1 oder 2, **dadurch gekennzeichnet, dass** der Zylinder (30) durch einen dichtend gegen den Ventilblock (26) anliegenden Zylindereinsatz (30) gebildet ist.

4. Pumpmodul nach Anspruch 3, **dadurch gekennzeichnet, dass** der Zylinder (30) an dem Ventilblock (26) befestigt ist.

5. Pumpmodul nach einem der vorherigen Ansprüche, **dadurch gekennzeichnet, dass** das Abdeckelement (28) unmittelbar mit dem Ventilblock (26) verbunden ist.

6. Pumpmodul nach einem der Ansprüche 3 bis 5, **dadurch gekennzeichnet, dass** der Zylindereinsatz (30) endseitig gegen eine in dem Ventilblock (26) vorgesehene Ventilbuchse (34) anliegt.

7. Pumpmodul nach einem der Ansprüche 3 bis 6, **gekennzeichnet durch** eine vormontierte Pumpeinheit (10) die den zumindest einen Zylindereinsatz (30), den Ventilblock (26) und das Abdeckelement (28) umfasst.

8. Pumpmodul nach einem der vorherigen Ansprüche, **gekennzeichnet durch** eine Gehäusebasis (2), die einen vorderen Ausgabebereich zur Aufnahme des Ventilblocks (26) und/oder einen hintere Antriebsbereich (20) zur Aufnahme des Zylinders (30) und/oder des Pumpkolbens (4) ausbildet.

9. Pumpmodul nach einem der vorherigen Ansprüche, **dadurch gekennzeichnet, dass** der in der Phasengrenze zwischen dem Abdeckelement (28) und dem Ventilblock (26) vorgesehene Einlasskanal (44) mit zumindest zwei Zylindern (30) kommuniziert und dass der Einlasskanal (44) innerhalb der Phasengrenze so ausgebildet ist, dass der Einlasskanal (44) den Auslasskanal (64) jedenfalls teilumfänglich umgibt.

10. Pumpmodul nach Anspruch 8 oder 9, **dadurch gekennzeichnet, dass** die Gehäusebasis (2), der Ventilblock (26) und das Abdeckelement (28) als Kunststoffteile ausgebildet sind.

11. Pumpmodul nach einem der Ansprüche 8 bis 10, **dadurch gekennzeichnet, dass** die Gehäusebasis (2) zu jedem Pumpkolben (4) eine dem Zylinder (30) vorgelagerte Führungsbuchse (76) ausbildet.

12. Pumpmodul nach Anspruch 11, **dadurch gekennzeichnet, dass** die Gehäusebasis (2) Führungs- und Verriegelungsflächen (22, 24) zur lösbaren Befestigung des Pumpmoduls an einem Antriebsgehäuse (112) eines Antriebs (110) ausbildet, dessen Antriebsstößel (124) mit dem Pumpkolben (4) zum reversierenden Betrieb des Pumpkolbens (4) verbindbar ist.

13. Pumpmodul nach einem der vorherigen Ansprüche, **gekennzeichnet durch** ein Kopfelement (8), das dem Abdeckelement (28) vorgelagert vorgesehen, mit einem Auslassanschluss (66) versehen und dicht gegen das Abdeckelement (28) angelegt ist.

14. Pumpmodul nach einem der vorherigen Ansprüche, **gekennzeichnet durch** ein an dem Gehäuse befestigtes Transponderelement (12).

15. Vorrichtung zur Erzeugung eines Flüssigkeitsstrahls, insbesondere für die Entfernung von biologischem Gewebe, mit einem Antriebsgehäuse (112), einem in dem Antriebsgehäuse (112) vorgesehenen Antrieb (110) und einem ein Pumpengehäuse (2, 8, 26, 28) aufweisenden Pumpmodul (120) nach einem der vorherigen Ansprüche, wobei das Antriebsgehäuse (112) und das Pumpmodul (120) lösbar fügbar sind und wobei der Antrieb (110) und das Pumpmodul (120) einander zugeordnete elektronische Kennungen aufweisen.

## Claims

1. Pump module with a pump casing (2, 8, 26, 28), a cylinder (30), at least one pump piston (4), which is mounted in the pump casing (2, 8, 26, 28) in a reciprocatingly movable manner and which is provided with at least one sealing element (32) that interacts with the cylinder (30) during the pumping operation, a valve block (26) that is sealed against the cylinder (30), at least one valve (37, 41) to said cylinder, said valve (37, 41) being received in the valve block (26), and a cover element (28) on a side opposite to said cylinder being in abutment against said valve block (26) and between itself and said valve block (26) forming an inlet passage (44) to said cylinder (30) and an outlet passage (64) communicating with said cylinder (30), **characterized in that** the inlet passage (44) and the outlet passage (64) each are formed by a recessed groove in the side of the valve block (26) opposite to the cylinder (30), the recessed groove being exposed toward the surface of the cover element (28), wherein the inlet passage (44) and the outlet passage (64) each are circumferentially closed by the interaction of the valve block (26) and the cover element (28) and extend at a right angle to the direction of motion of the reciprocatingly movable pump piston (4).

2. Pump module according to claim 1, **characterized in that** said valve block (26) comprises a port (18) for fluid to be conveyed in said pump.

3. Pump module according to claim 1 or 2, **characterized in that** said cylinder (30) is formed by a cylinder insert (30) abutting against said valve block (26) in a sealing manner.

4. Pump module according to claim 3, **characterized in that** said cylinder (30) is fastened to said valve block (26).

5. Pump module according to one of the preceding claims, **characterized in that** said cover element (28) is connected directly to said valve block (26).

6. Pump module according to one of the claims 3 to 5, **characterized in that** said cylinder insert (30) at the end side abuts against a valve liner (34) provided in said valve block (26).

7. Pump module according to one of the claims 3 to 6, **characterized by** a pre-assembled pump unit (10) comprising at least one cylinder insert (30), said valve block (26), and said cover element (28).

8. Pump module according to one of the preceding claims, **characterized by** a casing base (2) forming a front discharge region for receiving said valve block (26) and/or a rear drive region (20) for receiving said cylinder (30) and/or said pump piston (4).

9. Pump module according to one of the preceding claims, **characterized in that** said inlet passage (44) provided in the phase boundary between said cover element (28) and said valve block (26) is in communication with at least two cylinders (30), and that said inlet passage (44) is within the phase boundary formed such that said inlet passage (44) at least partially circumferentially surrounds said outlet passage (64).

10. Pump module according to claim 8 or 9, **characterized in that** said casing base (2), said valve block (26), and said cover element (28) are formed as plastic parts.

11. Pump module according to one of the claims 8 to 10, **characterized in that** the casing base (2) forms a guide sleeve (76) upstream of said cylinder (30) to each pump piston (4).

12. Pump module according to claim 11, **characterized in that** said casing base (2) forms guide and locking surfaces (22, 24) for detachably fastening said pump module to a drive casing (112) of a drive (110), the drive pusher (124) of which is connectable to said pump piston (4) for reciprocating operation of said pump piston (4).

13. Pump module according to one of the preceding claims, **characterized in that** a head element (8), which is provided upstream of said cover element (28), is provided with an outlet port (66) and is in tight abutment against said cover element (28).

14. Pump module according to one of the preceding claims, **characterized by** a transponder element (12) attached to said casing.

15. Device for producing a fluid jet, in particular for the removal of biological tissue, with a drive casing (112), a drive (110) provided in the drive casing (112) and a pump module (120) comprising a pump casing (2, 8, 26, 28) according to one of the previous claims, where said drive casing (112) and said pump module (120) are detachably joinable, and where said drive (110) and said pump module (120) have mutually assigned electronic identifications.

## Revendications

1. Module de pompe comprenant un carter de pompe (2, 8, 26, 28), un cylindre (30), au moins un piston de pompe (4), qui est monté mobile de manière réversible dans le carter de pompe et est muni d'au moins un élément d'étanchéité (32) interagissant avec le cylindre (30) lors d'un fonctionnement en mode pompe, un bloc de valves (26) étanche par rapport au cylindre (30), au moins une valve (37, 41) vers le cylindre, qui est logée dans le bloc de valves (26), et un élément de couvercle de recouvrement (28), qui s'appuie sur le bloc de valves (26) du côté opposé à celui où se trouve le cylindre, et qui forme entre lui-même et le bloc de valves (26), un canal d'entrée (44) conduisant au cylindre (30), et un canal de sortie (64) communiquant avec le cylindre (30), **caractérisé en ce que** le canal d'entrée (44) et le canal de sortie (64) sont formés chacun respectivement par une rainure, qui est ouverte vers la surface de l'élément de couvercle de recouvrement (28), dans le côté du bloc de valves (26), opposé à celui où se trouve le cylindre (30), et qui est fermée de manière périphérique par l'interaction du bloc de valves (26) et de l'élément de couvercle de recouvrement (28), et s'étend perpendiculairement à la direction de mouvement du piston de pompe.

2. Module de pompe selon la revendication 1, **caractérisé en ce que** le bloc de valves (26) présente un raccord de branchement (18) pour le fluide à refouler dans la pompe.

3. Module de pompe selon la revendication 1 ou la revendication 2, **caractérisé en ce que** le cylindre (30) est formé par un insert de cylindre (30) s'appuyant de manière étanche contre le bloc de valves (26).

4. Module de pompe selon la revendication 3, **caractérisé en ce que** le cylindre (30) est fixé au bloc de valves (26).

5. Module de pompe selon l'une des revendications précédentes, **caractérisé en ce que** l'élément de couvercle de recouvrement (28) est relié directement au bloc de valves (26).

6. Module de pompe selon l'une des revendications 3 à 5, **caractérisé en ce que** l'insert de cylindre (30) s'appuie, à son extrémité, contre une douille de valve (34) prévue dans le bloc de valves (26).

7. Module de pompe selon l'une des revendications 3 à 6, **caractérisé par** une unité de pompe (10) prémontée, qui comprend ledit au moins un insert de cylindre (30), le bloc de valves (26) et l'élément de couvercle de recouvrement (28) .

8. Module de pompe selon l'une des revendications précédentes, **caractérisé par** une base de carter (2), qui forme une zone avant de distribution destinée à recevoir le bloc de valves (26), et/ou une zone arrière d'entraînement (20) destinée à recevoir le cylindre (30) et/ou le piston de pompe (4).

9. Module de pompe selon l'une des revendications précédentes, **caractérisé en ce que** le canal d'entrée (44) prévu dans l'interface entre l'élément de couvercle de recouvrement (28) et le bloc de valves (26), communique avec au moins deux cylindres (30), et **en ce que** le canal d'entrée (44) est réalisé à l'intérieur de l'interface de manière à ce que le canal d'entrée (44) entoure dans tous les cas partiellement de manière périphérique, le canal de sortie (64).

10. Module de pompe selon la revendication 8 ou la revendication 9, **caractérisé en ce que** la base de carter (2), le bloc de valves (26) et l'élément de couvercle de recouvrement (28) sont réalisés sous forme de pièce de matière plastique.

11. Module de pompe selon l'une des revendications 8 à 10, **caractérisé en ce que** la base de carter (2) forme pour chaque piston de pompe (4), une douille de guidage (76) précédant le cylindre (30).

12. Module de pompe selon la revendication 11, **caractérisé en ce que** la base de carter (2) forme des surfaces de guidage et de verrouillage (22, 24) pour la fixation amovible du module de pompe à un carter d'entrainement (112) d'un entraînement (110), dont le poussoir d'entrainement (124) peut être relié au piston de pompe (4) pour le fonctionnement réversible du piston de pompe (4).

13. Module de pompe selon l'une des revendications précédentes, **caractérisé par** un élément de tête (8), qui est prévu au-devant de l'élément de couvercle de recouvrement (28), est muni d'un raccord de sortie (66) et est appliqué de manière étanche contre l'élément de couvercle de recouvrement (28).

14. Module de pompe selon l'une des revendications précédentes, **caractérisé par** un élément de transpondeur (12) fixé au carter.

15. Dispositif pour produire un jet de liquide, notamment pour l'évacuation de tissus biologiques, comprenant un carter d'entrainement (112), un entraînement (110) prévu dans le carter d'entrainement (112), et un module pompe (120) selon l'une des revendications précédentes et comprenant un carter de pompe (2, 8, 26, 28), le carter d'entrainement (112) et le module de pompe (120) pouvant être assemblés de manière démontable, et l'entrainement (110) et le module de pompe (120) présentant des identifiants électroniques mutuellement associés.
